# EUROPEAN PATENT APPLICATION

(11) **EP 4 335 375 A1**
(43) Date of publication of application: **13.03.2024**
(21) Application number: 22195127.0
(22) Date of filing: 12.09.2022
(51) Int. Cl.: A61B 6/02, A61B 6/03, A61B 6/00, F16F 15/00, F16F 15/30, G01N 23/046

(54) **MEDICAL VEHICLE AND METHOD FOR OPERATING A MEDICAL VEHICLE**

(71) Applicant: Koninklijke Philips N.V., 5656 AG Eindhoven (NL)
(72) Inventor: WEISS, Steffen, Eindhoven (NL); JOHNSON, Mark Thomas, Eindhoven (NL); VOGTMEIER, Gereon, 5656AG Eindhoven (NL)
(74) Representative: Philips Intellectual Property & Standards

(57) **Abstract**

The invention relates to a medical vehicle (1) comprising a base vehicle (2) and a compartment (3). The compartment (3) has a compartment room (5) and a medical imaging system (4) and is a part of the base vehicle (2) or is attached to the base vehicle (2). The medical imaging system (4) comprises at least one rotating component (6). The medical vehicle (1) further comprises a counter-rotating apparatus (7) with at least one rotor (8), wherein the counter-rotating apparatus (7) is configured to be operated such that the sum of the angular momentum of the rotating component(s) (6) and the angular momentum of the counter-rotating apparatus (7) is almost equal to or exactly equal to zero. The invention further relates to a base vehicle (2), a medical imaging system (4) and a method for operating said medical vehicle (1).

## Description

### FIELD OF THE INVENTION

The invention relates to a medical vehicle comprising a base vehicle and a compartment with a compartment room and a medical imaging system, wherein the medical imaging system comprises at least one rotating component. The invention further relates to a method for operating said medical vehicle.

### BACKGROUND OF THE INVENTION

Mobile computed tomography (CT) systems may be used in mobile stroke units to timely diagnose stroke and differentiate ischemic and hemorrhagic types. This enables to plan the correct form of treatment before arrival in the hospital and start treatment immediately after arrival in the hospital. Such mobile stroke units may be installed in stroke ambulances and trucks, on ships and helicopters and even in spacecraft.

During transportation, the imaging system can be used for some, at least low quality, imaging, in order to optimize the time and accessibility. But at the same time, the imaging system will be subjected to forces caused by the motion of the vehicle. During operation in a stationary setting, the associated forces are constant and as such are accounted for in the construction of the system. However, if a massive part of the system is rotating (as, e.g., when the CT or X-ray system is scanning while the vehicle is driving), additional non-constant forces are induced on the rotating parts: accelerative forces and especially gyroscopic torques and related forces. This is particularly the case for CT systems which have a massive and fast rotating gantry, here called CT gantry, and also a fast rotating anode in the X-ray tube. A typical CT gantry weighs about a ton and rotates at up to 4 Hz. The gyroscopic torques caused by a CT in a mobile vehicle can induce excessive forces on the support of the CT system. The magnitude and direction of the gyroscopic torque are related to the mass, mass distribution and rotation speed of the rotating component and the rate and direction at which the axis of the rotating component changes, which is in a vehicle related to the driving speed and curvature radius of the road.

The gyroscopic torque will therefore cause strain on the support and actually change the driving behavior of the vehicle. A rotating component which is forced to change direction of its rotation axis will break out perpendicularly to this force. If the rotation axis of the rotating component is along a longitudinal direction of the vehicle and the vehicle does a turn, the nose of the vehicle will be raised or lowered by a gyroscopic torque that is proportional to the angular speed of the rotating component, the moment of inertia of the rotating component and the angular speed of the vehicle driving around the curve.

In case of an accident or in case of sudden changes of driving direction as a sharp turn for a road vehicle, the rate at which the rotating component is forced to change direction may be particularly high, and therefore the torques and related forces may cause unforeseen motion of the entire vehicle. Moreover, the forces may exceed the mechanical limits of the support and cause the CT system to break out of its support.

### SUMMARY OF THE INVENTION

It is therefore an object of the present invention to provide a medical vehicle with an improved and safer ability to perform medical scans during operation of the medical vehicle. It is a further method of the present invention to provide a corresponding method for operating a medical vehicle.

The object of the present invention is solved by the subject matter of the independent claims, wherein further embodiments are incorporated in the dependent claims.

In an aspect of the present invention, a medical vehicle comprising a base vehicle and a compartment is provided, wherein the compartment comprises a compartment room and a medical imaging system. The main purpose of the medical vehicle may be the medical imaging, i.e., performing medical scans using the medical imaging system. Here, the medical vehicle may be optimized to perform medical scans during motion of the medical vehicle, and/or it may be optimized to perform medical scans when the medical vehicle is stopped while being configured to perform medical scans during motion of the medical vehicle. Alternatively, or additionally, the main purpose of the medical vehicle may be a non-medical one, such as a cruise ship that has a medical imaging system as part of its medical equipment.

The base vehicle is the vehicle transporting the compartment with the compartment room and the medical imaging system, i.e., the base vehicle is the medical vehicle without the compartment. As above, the base vehicle may be specifically configured for the use with the medical imaging system, such as a truck with a medical imaging system, and/or the base vehicle may have a non-medical main purpose, such as a cruise ship.

The compartment is a part of the base vehicle or is attached to the base vehicle. In other words, the compartment may be a virtual compartment defined by a volume of an existing compartment of the base vehicle or may comprise physical walls. In the latter case, the compartment may be permanently attached to the base vehicle or may be detachable such that it can be placed separately from the base vehicle and/or that a different compartment may be attached to the base vehicle. The compartment may be just large enough to fit the medical imaging system, such that the compartment room is very small. The compartment may also provide a larger compartment room, e.g., to provide extra space next to the medical imaging system, for example for medical staff.

The medical imaging system comprises at least one rotating component. Said rotating component(s) may be configured to rotate around a rotation axis. The medical imaging system may further comprise a bearing that supports the rotating component(s) and many other components that depend on the exact type of medical imaging system.

The medical vehicle further comprises a counter-rotating apparatus with at least one rotor. Said rotor(s) may be configured to rotate around a rotation axis. The counter-rotating apparatus is configured to be operated such that the sum of the angular momentum of the rotating component(s) and the angular momentum of the counter-rotating apparatus is almost equal to or exactly equal to zero. Said operation of the counter-rotating apparatus may be performed by controlling an angular velocity of the rotor(s) of the counter-rotating apparatus. As an example, the moment of inertia of the rotating component(s) and the rotor(s) as well as the angular velocity of the rotating component(s) may be known and then the angular velocity of the rotor(s) needed such that the angular momentum of the rotor(s) compensates the angular momentum of the rotating component(s) may be easily computed. In this context, "almost equal to zero" means a significant reduction of the overall angular momentum, e.g., the sum of the angular momenta of the rotating component(s) and the counter-rotating apparatus may be less than 10 % of the angular momentum of the rotating component(s), preferably less than 5 % of the angular momentum of the rotating component(s), most preferably less than 1 % of the angular momentum of the rotating component(s).

Hence, the angular momentum of the rotating component(s) is compensated, and the net angular momentum is reduced (or neutralized) to enable medical imaging during transport and to provide safe medical imaging during transport. In particular, gyroscopic forces that may arise when the medical imaging system is rotated, e.g., when driving around a curve, are minimized.

According to an embodiment, the base vehicle is a truck, a train, a plane, a helicopter, a spacecraft, an autonomous flight object and/or a ship. In all of said vehicles, a medical imaging system may be installed, transported and used during operation of the medical vehicle. Rotations of the medical imaging system may stem from, e.g., curves on the route, potholes, hills, turbulences, waves or an uncontrolled motion during accidents.

According to an embodiment, the medical imaging system is a computed tomography, CT, system, a positron emission tomography-computed tomography, PET-CT, system or an X-ray system. However, the medical imaging system may be any medical imaging system that comprises rotating components. In the case of a CT system or a PET-CT system, one rotating component is a CT gantry and another rotating component may be an X-ray anode. In the case of the X-ray system, one rotating component is the X-ray anode. In the case of the CT system or PET-CT system, the angular momentum of the CT gantry may be greater than the angular momentum of the X-ray anode, i.e., the overall angular momentum of the rotating components may be dominated by the CT gantry. In this case, the rotor(s) of the counter-rotating apparatus may rotate in an opposite direction to the CT gantry. Also, since the angular momentum of the X-ray anode may be smaller than the angular momentum of the CT gantry, the gyroscopic forces and torques caused by the X-ray anode may be smaller than those of the CT gantry and one or more rotor(s) compensating the combined angular momentum of the CT gantry and the X-ray anode may be sufficient. In particular, if the rotation axis of the X-ray anode is parallel to the rotation axis of the CT gantry, the rotation axis of the rotor(s) may also be parallel to the rotation axis of the CT gantry. The rotation speed of the rotor(s) may then be controlled based on the known moment of inertia of the CT gantry and the X-ray anode as well as on their respective angular velocities.

According to an embodiment, a moment of inertia of at least one of the rotors is adjustable. Said adjustment may be performed, in particular, by changing the distance of masses of the rotor from the rotation axis. Operating the counter-rotating apparatus such that the sum of the angular momentum of the rotating component(s) and the angular momentum of the counter-rotating apparatus is almost equal to or exactly equal to zero may then be performed by adjusting the moment of inertia of at least one of the rotor(s) while rotating them at a predetermined angular velocity or by both adjusting the moment of inertia of at least one of the rotors and controlling the angular velocity of the rotor(s).

According to an embodiment, at least one of the rotors is allocated to the base vehicle and/or to the compartment room. That is, said rotor is not part of the medical imaging system. A rotor allocated to the base vehicle is particularly useful when the base vehicle is adapted to be used with different medical imaging systems. Then, said rotor may be used to compensate the angular momentum of any of said medical imaging systems. A rotor allocated to the compartment room, but not to the medical imaging system, may be used when it is too difficult and/or too expensive to equip the medical imaging system with such a rotor.

According to an embodiment, a rotation axis of at least one of the rotors is adjustable. The rotation axis may then be adjusted to match different rotation axes (or effective rotation axes) of different medical imaging systems, in particular, when the base vehicle is configured to be used with different medical imaging systems. Alternatively, or additionally, the rotation axis may be adjusted when the (effective) rotation axis of the rotating component(s) changes, e.g., when the CT gantry is tilted.

Alternatively, or additionally, there may be two or more rotors that have rotation axes that are not parallel to one another, in particular that are perpendicular to one another. Then, both direction and magnitude of the angular momentum of the counter-rotating apparatus can be adjusted by controlling the angular velocities of these rotors.

According to an embodiment, at least one of the rotors is allocated to the medical imaging system. Hence, the compensation of the angular momentum may be performed within the medical imaging system and the components of the medical vehicle outside the medical imaging system may not be subjected to gyroscopic forces or torques when the medical vehicle makes a rotational motion.

According to an embodiment, at least one of the rotors is an active imaging rotor. As an example, two identical CT gantries may be configured adjacent to each other and driven so as to rotate in opposite directions. In this case, one of the CT gantries is considered the rotating component and the other one is considered the rotor. If the two CT gantries are rotated at the same angular velocity, the net angular momentum is zero. As another example, a first CT gantry may be sandwiched by two smaller CT gantries, wherein the smaller CT gantries are rotated in an opposite direction to the first CT gantry. A net angular momentum of zero may be achieved, e.g., when the smaller CT gantries each have half the mass of the first CT gantry and are rotated at the same angular velocity.

According to an embodiment, at least one of the rotors is a ring-layer and is arranged outside of, adjacent to and coaxial with one of the rotating components. Since said rotor has a larger radius than the rotating component that is surrounded by the rotor, a comparably lower mass of the rotor may be sufficient to achieve a moment of inertia that is large enough such that the angular momentum of the rotating component may be compensated at reasonable angular velocities. Also, in particular when a material with a high density is used for the rotor, this may lead to a thin rotor. As an example, the rotor may be a thin ring surrounding a CT gantry.

According to an embodiment, at least one of the rotors is arranged on an axial side of one of the rotating components. Said rotor(s) may be arranged on the same rotation axis as the rotating component. In particular, two of the rotors are arranged symmetrically, one on each axial side of one of the rotating components. Hence, said rotating component is sandwiched by the rotors. As an example, two rotors may be arranged on each side of a CT gantry.

When the rotating component(s) and the rotor(s) share a rotation axis, acceleration and/or deceleration of the rotating component(s) and the rotor(s) may be performed against one another, i.e., the torque to accelerate or decelerate the rotating component(s) is exerted on the rotor(s) and vice versa. Hence, only little or even no torque is exerted on the vehicle.

In another aspect of the present invention, a base vehicle for a medical vehicle is provided. The medical vehicle comprises a compartment for a medical imaging system, or the base vehicle is adapted to be connected with a compartment with a medical imaging system. The medical imaging system comprises at least one rotating component and the base vehicle comprises a counter-rotating apparatus with at least one rotor. The counter-rotating apparatus is configured to be operated such that the sum of the angular momentum of the rotating component(s) and the angular momentum of the counter-rotating apparatus is almost equal to or exactly equal to zero. Hence, the base vehicle may be used with different medical imaging systems and the counter-rotating apparatus compensates the angular momentum of the medical imaging system, such that the angular momentum of the medical imaging system does not adversely affect the motion of the base vehicle. The base vehicle may be connected to the medical imaging system, e.g., by a wired or wireless connection, to receive data related to the angular momentum of the medical imaging system, e.g., the angular velocity of the rotating components, such that the angular velocity of the rotors may be controlled to compensate the angular momentum. Further details and advantages are given in the above description of the medical vehicle.

In yet another aspect of the present invention, a medical imaging system for a medical vehicle is provided. The medical imaging system comprises at least one rotating component and a counter-rotating apparatus with at least one rotor. The counter-rotating apparatus is configured to be operated such that the sum of the angular momentum of the rotating component(s) and the angular momentum of the counter-rotating apparatus is almost equal to or exactly equal to zero. Hence, the medical imaging system may be used in the medical vehicle without adversely affecting the motion of the medical vehicle due to gyroscopic forces. Also, due to the cancellation of the angular momentum, safety of the medical imaging system is increased. Further details and advantages are given in the above description of the medical vehicle.

In yet another aspect of the present invention, a method for operating a medical vehicle according to the above description is provided. According to the method, an angular momentum of the rotating component(s) is determined, and an angular velocity of the rotor(s) is controlled such that the sum of the angular momentum of the rotating component(s) and the angular momentum of the counter-rotating apparatus is almost equal to or exactly equal to zero. The determination of the angular momentum of the rotating component(s) may be performed by providing the moment of inertia of the rotating component(s), e.g., by user input, and by determining, e.g., measuring, the angular velocity of the rotating component(s). Further, the moment of inertia of the rotor(s) may be provided, e.g., by user input. With this information, the angular velocity of the rotor(s) needed to compensate the angular momentum of the rotating component(s) may be easily computed. Further details and advantages are given in the above description of the medical vehicle.

According to an embodiment, the angular velocity of the rotor(s) is controlled dynamically. In particular, the angular velocity of the rotating component(s) may be determined periodically, and the angular velocity of the rotor(s) adjusted accordingly.

According to an embodiment, controlling of the angular velocity of the rotor(s) is synchronized with controlling an angular velocity of the rotating component(s). As an example, control information provided to change the angular velocity of the rotating component(s), e.g., when a scan is started or stopped, may be directly provided to the counter-rotating apparatus such that the control of the angular velocity of the rotor(s) may be synchronized with the control of the angular velocity of the rotating component(s). As another example, control information provided by the base vehicle that leads to a change of the angular velocity of the rotating component(s), e.g., a reduction of angular velocity due to upcoming curvy roads, may be directly provided to the counter-rotating apparatus. As yet another example, control information indicating an accident may be provided by the base vehicle, such that the rotating component(s) and the rotor(s) shall be stopped synchronized to one another and as rapidly as possible. This is particularly efficient when the deceleration of the rotating component(s) is performed against the rotor(s) and vice versa. Hence, breaking out of the rotating component(s), e.g., the CT gantry, and the rotor(s) out of their bearings may be prevented.

It shall be understood that a preferred embodiment of the invention can also be any combination of the dependent claims with the respective independent claim.

These and other aspects of the invention will be apparent from and elucidated with reference to the embodiments described hereinafter.

### BRIEF DESCRIPTION OF THE DRAWINGS

In the following, preferred embodiments of the invention will be described, by way of example only, and with reference to the drawings in which:
Fig. 1a shows a longitudinal cross-section through an embodiment of a medical vehicle;
Fig. 1b shows a transverse cross-section through the medical imaging system of the medical vehicle of Fig. 1a;
Fig. 2 shows a longitudinal cross-section through another embodiment of a medical vehicle;
Fig. 3 shows a longitudinal cross-section through yet another embodiment of a medical vehicle;
Fig. 4 shows a longitudinal cross-section through yet another embodiment of a medical vehicle;
Fig. 5 shows a longitudinal cross-section through yet another embodiment of a medical vehicle;
Fig. 6 shows a longitudinal cross-section through an embodiment of a base vehicle; and
Fig. 7 shows a longitudinal cross-section through a medical imaging system.
In the figures, elements which correspond to elements already described may have the same reference numerals. Examples, embodiments or optional features, whether indicated as non-limiting or not, are not to be understood as limiting the invention as claimed.

### DETAILED DESCRIPTION OF EMBODIMENTS

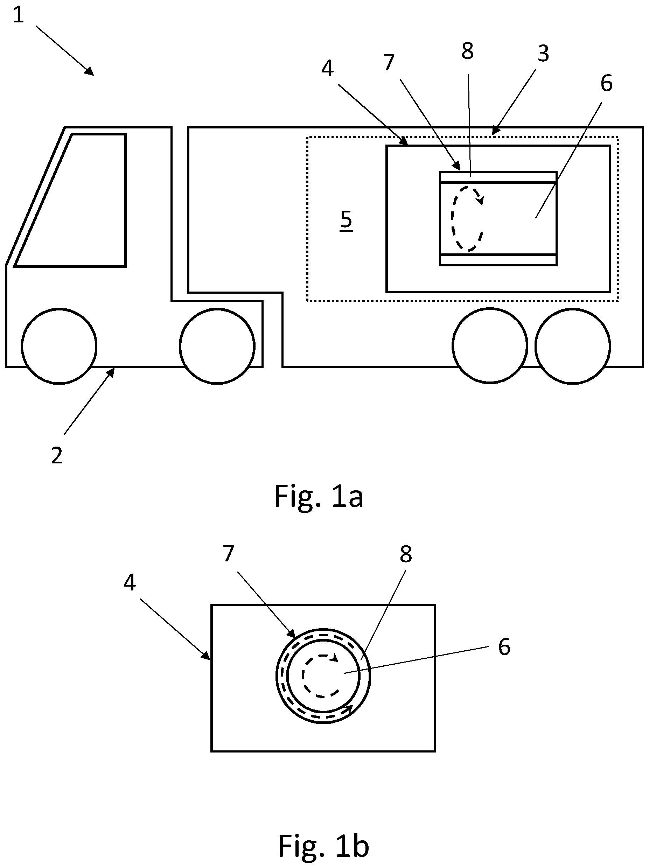

Fig. 1a shows a schematic longitudinal cross-section through a medical vehicle 1. The medical vehicle 1 comprises a base vehicle 2. While the base vehicle 2 is shown as a truck, the base vehicle 2 may as well be a train, a plane, a helicopter, a spacecraft, an autonomous flight object and/or a ship.

The medical vehicle 1 further comprises a compartment 3 with a medical imaging system 4 and compartment room 5. The compartment 3 may be a part of the base vehicle 2 or may be attached to the base vehicle 2. In other words, the compartment 3 may be a virtual compartment defined by a volume of an existing compartment of the base vehicle 2 or may comprise physical walls. In the latter case, the compartment 3 may be permanently attached to the base vehicle 2 or may be detachable such that it can be placed separately from the base vehicle 2 and/or that a different compartment 3 may be attached to the base vehicle 2. The compartment 3 may be just large enough to fit the medical imaging system 4, such that the compartment room 5 is very small. The compartment 3 may also provide a larger compartment room 5, e.g., to provide extra space next to the medical imaging system 4, for example for medical staff.

The main purpose of the medical vehicle 1 may be the medical imaging, i.e., performing medical scans using the medical imaging system 4. Here, the medical vehicle 1 may be optimized to perform medical scans during operation of the medical vehicle 1, and/or it may be optimized to perform medical scans when the medical vehicle 1 is stopped while being configured to perform medical scans during operation of the medical vehicle 1. Alternatively, or additionally, the main purpose of the medical vehicle 1 may be a non-medical one, such as a cruise ship that has a medical imaging system 4 as part of its medical equipment.

The medical imaging system 4 comprises at least one rotating component 6. The medical imaging system 4 may be a computed tomography, CT, system, a positron emission tomography-computed tomography, PET-CT, system or an X-ray system. However, the medical imaging system 4 may be any medical imaging system that comprises rotating components 6. In the case of a CT system or a PET-CT system, one rotating component 6 is a CT gantry and another rotating component may be an X-ray anode. In the case of the X-ray system, one rotating component is the X-ray anode. A transverse cross-section through the medical imaging system 4 is shown in Fig. 1b.

The medical vehicle 1 further comprises a counter-rotating apparatus 7 comprising at least one rotor 8. In the present embodiment, the rotor 8 is a thin ring-layer that is arranged outside of, adjacent to and coaxial with the rotating component 6.

The counter-rotating apparatus 7 is configured to be operated, in particular by controlling an angular velocity of the rotor 8, such that the sum of the angular momentum of the rotating component 6 and the angular momentum of the counter-rotating apparatus 7, i.e., the angular momentum of the rotor 8, is almost equal to or exactly equal to zero. In this context, "almost equal to zero" means a significant reduction of the overall angular momentum, e.g., the sum of the angular momenta of the rotating component 6 and the counter-rotating apparatus 7 may be less than 10 % of the angular momentum of the rotating component 6, preferably less than 5 % of the angular momentum of the rotating component 6, most preferably less than 1 % of the angular momentum of the rotating component 6. In order to achieve said compensation of the angular momentum, the rotor 8 has to be rotated in a direction opposite to the rotation of the rotating component 6, as indicated by the dashed arrows.

Hence, the angular momentum of the rotating component 6 is compensated and the net angular momentum is reduced (or neutralized) to enable medical imaging during transport and to provide safe medical imaging during transport. In particular, gyroscopic forces that may arise when the medical imaging system is rotated, e.g., when driving around a curve, are minimized.

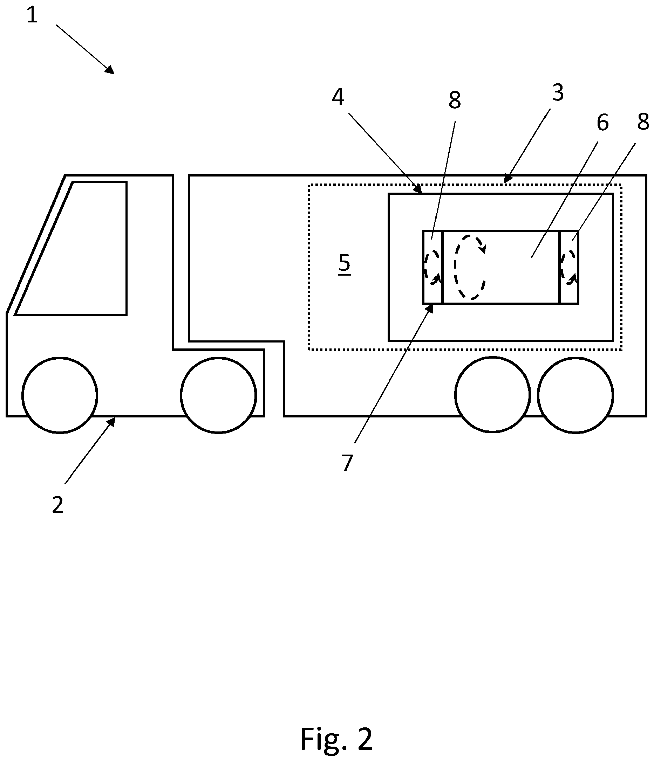

Fig. 2 shows a cross-section of another embodiment of a medical vehicle 1. In this embodiment, two rotors 8 of the counter-rotating apparatus 7 are arranged symmetrically, one on each axial side of the rotating component 6. In order to provide a clearance for a patient and in order to achieve the highest ratio of moment of inertia per mass, the rotors 8 may be ring-type rotors. In another embodiment (not shown here), just one rotor 8 may be arranged on one axial side of the rotating component (6).

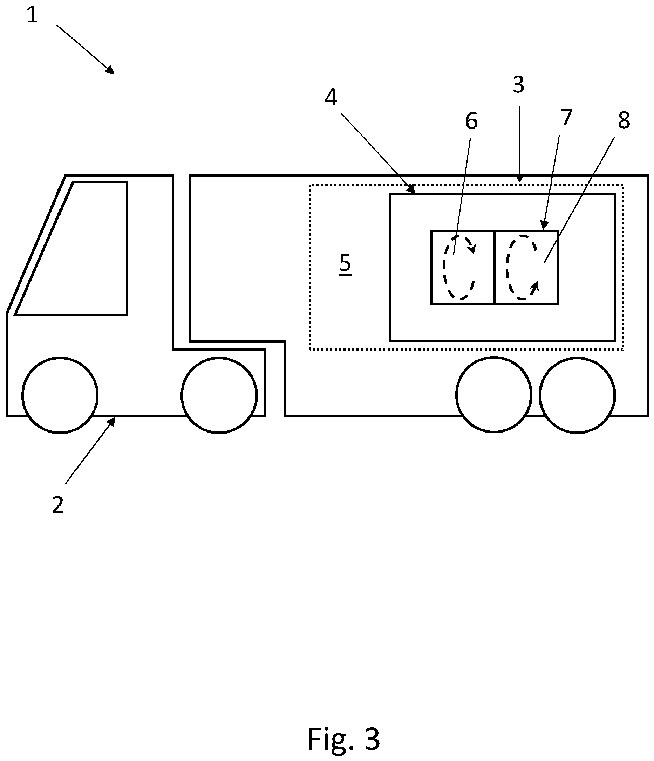

Fig. 3 shows a cross-section of yet another embodiment of a medical vehicle 1. In this embodiment, the rotor 8 is an active imaging rotor that is arranged at one axial side of the rotating component 6. As an example, the rotor 8 may also be a CT gantry and the rotating component 6 and the rotor 8 alternately take images in order not to disturb one another.

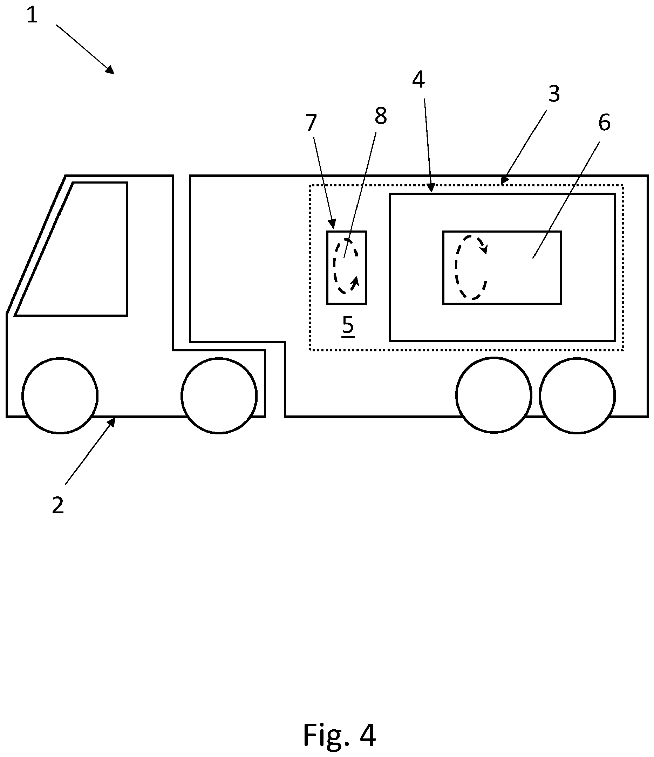

Fig. 4 shows a cross-section of yet another embodiment of a medical vehicle 1. In this embodiment, the counter-rotating apparatus 7 is arranged inside the compartment room 5. The information about the angular velocity of the rotating component 6 may be transmitted to the counter-rotating apparatus 7 via a wired or wireless connection. This placement of the counter-rotating apparatus 7 may be made when the medical imaging system 4 does not provide enough space to fit the rotor 8 or when the counter-rotating apparatus 7 is retrofitted to the medical imaging system 1. If the rotation axis of the rotating component 6 can be adjusted, e.g., tilted, the rotation axis of the rotor 8 should also be adjustable.

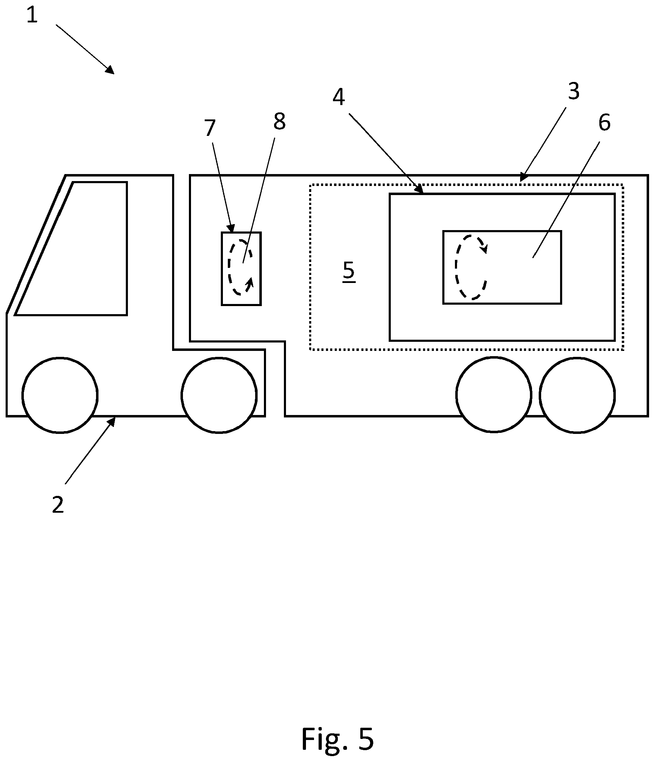

Fig. 5 shows a cross-section of yet another embodiment of a medical vehicle 1. In this embodiment, the counter-rotating apparatus 7 is allocated to the base vehicle 2. This is particularly useful when different medical imaging systems 4 may be installed on the base vehicle 2 and a compensation of angular momentum shall be performed for each of said medical imaging systems 4. Like in the previous embodiment, an adjustable rotation axis of the rotor 8 allows an adaptation to different rotation axes of the rotating components 6.

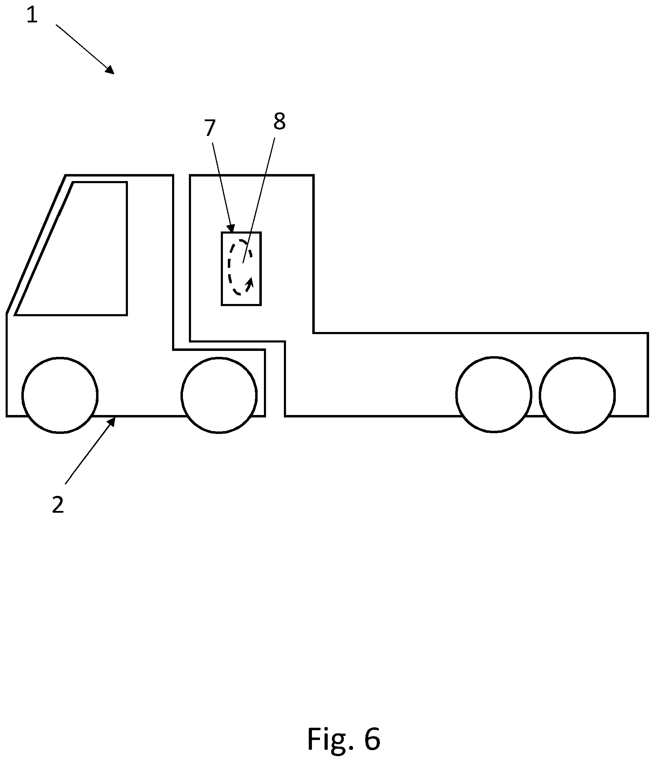

Fig. 6 shows a cross-section of a base vehicle 2. Said base vehicle 2 is adapted to be connected to different medical imaging systems and comprises a counter-rotating apparatus 7 that is adapted to compensate the angular momentum of said medical imaging systems. As above, an adjustable rotation axis of the rotor 8 allows an adaptation to different rotation axes of the rotating components 6.

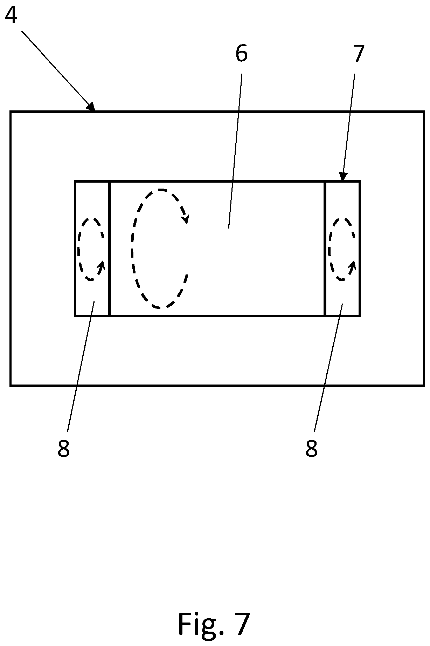

Fig. 7 shows a cross-section of a medical imaging system 4 with a rotating component 6 and a counter-rotating apparatus 7. Here, the rotors 8 of the counter-rotating apparatus 7 are arranged symmetrically, one on each axial side of the rotating component 6, but other arrangements are possible. When the rotors 8 share a rotation axis with the rotating component 6, gyroscopic forces that are exerted on the bearings that support the rotation axis when the medical imaging system 4 is rotated may be reduced.

While the invention has been illustrated and described in detail in the drawing and foregoing description, such illustration and description are to be considered illustrative or exemplary and not restrictive; the invention is not limited to the disclosed embodiments.

Other variations to the disclosed embodiments can be understood and effected by those skilled in the art in practicing the claimed invention, from a study of the drawings, the disclosure, and the appended claims. In the claims, the word "comprising" does not exclude other elements or steps, and the indefinite article "a" or "an" does not exclude a plurality. The mere fact that certain measures are recited in mutually different dependent claims does not indicate that a combination of these measured cannot be used to advantage. Any reference signs in the claims should not be construed as limiting the scope.

### LIST OF REFERENCE SIGNS:

- 1: medical vehicle
- 2: base vehicle
- 3: compartment
- 4: medical imaging system
- 5: compartment room
- 6: rotating component
- 7: counter-rotating apparatus
- 8: rotor

## Claims

1. Medical vehicle (1) comprising a base vehicle (2) and a compartment (3) with a compartment room (5) and a medical imaging system (4), wherein the compartment (3) is a part of the base vehicle (2) or the compartment (3) is attached to the base vehicle (2) and the medical imaging system (4) comprises at least one rotating component (6), and wherein the medical vehicle (1) further comprises a counter-rotating apparatus (7) with at least one rotor (8), wherein the counter-rotating apparatus (7) is configured to be operated such that the sum of the angular momentum of the rotating component(s) (6) and the angular momentum of the counter-rotating apparatus (7) is almost equal to or exactly equal to zero.

2. Medical vehicle (1) according to claim 1, wherein the base vehicle (2) is a truck, a train, a plane, a helicopter, a spacecraft, an autonomous flight object and/or a ship.

3. Medical vehicle (1) according to claim 1 or 2, wherein the medical imaging system (4) is a computed tomography, CT, system, a positron emission tomography-computed tomography, PET-CT, system or an X-ray system and the rotating component (6) is a CT gantry and/or an X-ray anode.

4. Medical vehicle (1) according to any of claims 1 to 3, wherein a moment of inertia of at least one of the rotors (8) is adjustable.

5. Medical vehicle (1) according to any of claims 1 to 4, wherein at least one of the rotors (8) is allocated to the base vehicle (2) and/or to the compartment room (5).

6. Medical vehicle (1) according to claim 5, wherein a rotation axis of at least one of the rotors (8) is adjustable.

7. Medical vehicle (1) according to any of claims 1 to 6, wherein at least one of the rotors (8) is allocated to the medical imaging system (4).

8. Medical vehicle (1) according to claim 7, wherein at least one of the rotors (8) is an active imaging rotor.

9. Medical vehicle (1) according to claim 7 or 8, wherein at least one of the rotors (8) is a ring-layer and is arranged outside of, adjacent to and coaxial with one of the rotating components (6).

10. Medical vehicle (1) according to any of claims 7 to 9, wherein at least one of the rotors (8) is arranged on an axial side of one of the rotating components (6), in particular two of the rotors (8) are arranged symmetrically, one on each axial side of one of the rotating components (6).

11. Base vehicle (2) for a medical vehicle (1), wherein the medical vehicle (1) comprises a compartment (3) for a medical imaging system (4) or the base vehicle (2) is adapted to be connected with a compartment (3) with a medical imaging system (4), wherein the medical imaging system (4) comprises at least one rotating component (6) and the base vehicle (2) comprises a counter-rotating apparatus (7) with at least one rotor (8), wherein the counter-rotating apparatus (7) is configured to be operated such that the sum of the angular momentum of the rotating component(s) (6) and the angular momentum of the counter-rotating apparatus (7) is almost equal to or exactly equal to zero.

12. Medical imaging system (4) for a medical vehicle (1), wherein the medical imaging system (4) comprises at least one rotating component (6) and a counter-rotating apparatus (7) with at least one rotor (8), wherein the counter-rotating apparatus (7) is configured to be operated such that the sum of the angular momentum of the rotating component(s) (6) and the angular momentum of the counter-rotating apparatus (7) is almost equal to or exactly equal to zero.

13. Method for operating a medical vehicle (1) according to any of claims 1 to 10, wherein an angular momentum of the rotating component(s) (6) is determined and an angular velocity of the rotor(s) (8) is controlled such that the sum of the angular momentum of the rotating component(s) (6) and the angular momentum of the counter-rotating apparatus (7) is almost equal to or exactly equal to zero.

14. Method according to claim 13, wherein the angular velocity of the rotor(s) (8) is controlled dynamically.

15. Method according to claim 14, wherein controlling of the angular velocity of the rotor(s) (8) is synchronized with controlling an angular velocity of the rotating component(s) (6).
